Europäisches Patentamt

European Patent Office

Office européen des brevets

(11) Numéro de publication: **0 095 432**

**A1**

## (12) DEMANDE DE BREVET EUROPEEN

(21) Numéro de dépôt: **83450014.2**

(22) Date de dépôt: **24.05.83**

(51) Int. Cl.³: **C 12 Q 1/26**
**G 01 N 33/14**

(30) Priorité: **25.05.82 FR 8209458**

(43) Date de publication de la demande:
**30.11.83 Bulletin 83/48**

(84) Etats contractants désignés:
**AT BE CH DE FR GB IT LI LU NL SE**

(71) Demandeur: **SOCIETE TECHNOLOGIE DIFFUSION
FRANCE S.A.R.L. T.D.F.
Bordeneuve St. Martin le Vieil
F-11170 Alzonne(FR)**

(71) Demandeur: **INSTITUT COOPERATIF DU VIN I.C.V.
18 av. F. Mistral
F-34000 Montpellier(FR)**

(71) Demandeur: **ECOLE NATIONALE SUPERIEURE
D'AGRONOMIE DE MONTPELLIER E.N.S.A.M.
Place Viala
F-34000 Montpellier(FR)**

(72) Inventeur: **Jalbert, Paul
14 Route de Laure
F-11800 Trebes(FR)**

(72) Inventeur: **Pineau, Jean
36 Boulevard des Arceaux
F-34000 Montpellier(FR)**

(72) Inventeur: **Salgues, Michel
Ensam
F-34000 Montpellier(FR)**

(74) Mandataire: **Ravina, Bernard
24, boulevard Riquet
F-31000 Toulouse(FR)**

(54) Procédé de détermination et de mesure de l'état sanitaire d'un matériau biologique et installation à cet effet.

(57) La présente invention a pour objet un procédé de mesure de l'état sanitaire d'un matériau biologique tel que par exemple de la vendange.

La présente invention a également pour objet l'installation mettant en oeuvre le procédé.

Le procédé de mesure de l'état sanitaire d'un matériau biologique selon l'invention se caractérise essentiellement en ce qu'il est fait usage de l'aptitude du parasite à générer par évolution de l'état sanitaire du matériau une oxydase utilisée comme traceur de l'état sanitaire apte à fixer l'oxygène sur un réactif adapté en ce qu'un échantillon du dit matériau est mis en contact d'une dose du dit réactif et en ce que après réaction par mesure de la quantité d'oxygène consommée par unité de temps et unité de volume est déterminée la présence ou l'absence de la dite oxydase et donc l'état sanitaire du matériau

Application : La mesure d'une activité oxydasique sur des matériaux biologiques, par exemple sur la vendange pour déterminer l'attaque du botrytis cinérea ou pourriture grise, l'oxydase étant en l'espèce la laccase.

./...

Fig.1

**0095432**

La présente invention a pour objet un procédé de détermination et de mesure de l'état sanitaire d'un matériau biologique.

Par état sanitaire d'un matériau biologique il y a lieu de comprendre toute altération et évolution liée au développement des champignons parasites, en particulier du botrytis cinéra et toute modification du Pool enzymatique oxydasique du matériau sous l'influence du parasite.

La présente invention a également pour objet une installation de mise en oeuvre d'un procédé tel qu'énoncé ci-dessus.

Il est important de pouvoir déterminer et de pouvoir mesurer l'état sanitaire d'un matériau biologique tel que par exemple du mout du vendange.

Les procédés connus à ce jour relèvent essentiellement de méthodes empiriques.

La présente invention vise à procurer un procédé fiable de détermination de l'état sanitaire d'un matériau et de mesure de celui-ci.

La présente invention vise en outre à procurer une installation ou un appareillage capable de fonctionner de manière rapide et automatisée sur le terrain et ne necessitant pas de filtration.

A cet effet le procédé de détermination et de mesure de l'état sanitaire tel que définit plus haut, d'un matériau biologique selon l'invention se caractérise essentiellement en ce qu'il est fait usage de l'aptitude du parasite à générer par évolution de l'état sanitaire du matériau une oxydase utilisée comme traceur, révélateur de l'état sanitaire, et apte à fixer l'oxygène sur un réactif et en ce que un échantillon du dit matériau est mis en contact d'une dose du dit réactif en présence d'air ou d'un autre gaz et en ce que par mesure de la quantité d'oxygène consommée par unité de temps et de volume est déterminée l'absence ou la présence et la quantité de la dite oxydase et donc l'état sanitaire du matériau.

Suivant une forme préférée de réalisation de l'invention, l'oxydase et le réactif correspondant susceptible de révéler sa présence et de mesurer la quantité d'oxydase sont déterminés en fonction de la nature de l'affection sanitaire à détecter.

Suivant une autre caractéristique de l'invention l'installation de mesure permettant la mise en oeuvre du procédé mentionné, ci-dessus comporte au moins une cuve de mesure dans laquelle s'effectue la

réaction, dotée de moyens de mesure en relation avec une centrale de mesure de la consommation d'oxygène,et donc de l'activité oxydasique, la dite cuve étant dotée d'un dispositif de vidange commandé, au moins un dispositif de prise et de distribution d'une quantité du matériau sur lequel la mesure est effectuée, le dit dispositif étant en relation avec la dite cuve, au moins un dispositif de prise et de distribution d'une dose de réactif, le dit dispositif étant également en relation avec la dite cuve de mesure, l'ensemble des dits dispositif de prise de matériau, de réactif et de vidange étant commandés par une centrale électronique de commande et de traitement des données.

D'autres avantages et caractéristiques de l'invention apparaitrons à la lecture de la description, ci-après d'une forme préférée de réalisation donnée à titre d'exemple non limitatif et illustrée par les dessins joints en lesquels :

- La figure 1 est un graphisme comparatif illustrant la consommation d'oxygène dans le cas de mout de vendange, exemple choisi, sains et contaminés.

- La figure 2 est une vue schématique de l'installation à cet effet.

Le procédé selon l'invention de détermination et de mesure de l'état sanitaire d'un matériau biologique est fondé sur le fait qu'il est fait usage de l'aptitude du parasite à générer par évolution de l'état sanitaire du matériau une oxydase utilisée comme traceur, c'est-à-dire comme révélateur de l'état sanitaire, la dite oxydase étant apte à fixer l'oxygène sur un réactif en sorte que par mesure de la consommation d'oxygène est mesurée l'activité oxydasique.

Par mise en contact d'un échantillon du dit matériau de volume déterminé avec un réactif adapté, il est possible dans le cas où le matériau présente la dite oxydase d'en déceler la présence et l'importance par mesure de l'activité oxydasique, c'est-à-dire de la consommation d'oxygène qui est fixée sur le dit réactif dans un temps de réaction déterminé.

Comme il sera énoncé plus avant, la mesure de l'oxygène est obtenue par mesure d'intensité au moyen d'une électrode spécifique à oxygène.

L'exemple de mise en oeuvre du procédé selon l'invention, qui suit décrit son application à la détermination et la mesure de l'état sanitaire du mout de vendange par rapport à la pourriture grise c'est-à-dire au Botrytis cinera, mais il va de soi que la présente invention peut s'appliquer sans sortir du cadre du présent brevet à la mesure de la consommation d'oxygène et donc de l'activité oxydasique tous types de matériaux biologiques, alimentaires ou non, purifiés ou non, pateux ou liquides, ne necessitant pas de filtration.

Le Botrytis cinera, en abrégé BC, est une maladie de la vendange qui a d'importantes conséquences au niveau oenologique.

Il est donc necessaire au niveau des apports de vendange en cave de vinification de pouvoir déceler la présence du BC en sorte de séparer les lots sains des lots atteints qui demandent un traitement particulier curatif ou spécifique de la vendange.

Le Botrytis est un champignon générateur de la pourriture grise qui contient une oxydase spécifique, la laccose, qui est un enzyme apte à fixer l'oxygène sur un réactif constitué de ou comportant des polyphénols ou d'autres composés directement ou par oxydasion couplée dans laquelle le dit réactif n'interviendraient que comme intermédiaires d'oxydasion.

Le procédé selon l'invention utilise cette propriété en vue de déterminer l'activité oxydasique par la consommation d'oxygène résultant de l'attaque du Botrytis.

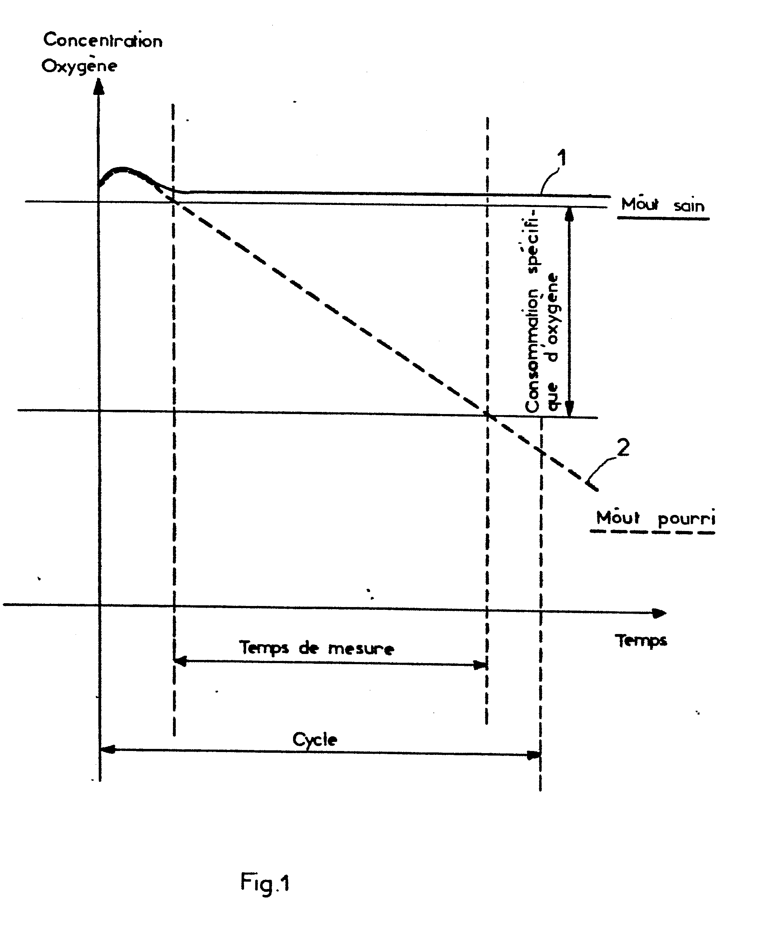

A cet effet, un volume déterminé de mout de vendange est prélevé et est mélangé à une quantité déterminée d'une dose d'un réactif en présence d'air ou d'un autre gaz, le milieu réactionnel subissant pendant la réaction une mesure de de la quantité d'oxygene consommée ce qui permet de déterminer l'activité oxydasique et donc la consommation d'oxygène par unité de temps et unité de volume du mout qui se traduit comme représenté à la fig.1 (graphique) dans laquelle la concentration spécifique d'oxygène du mout sain est représenté en 1 et la concentration spécifique d'oxygène du moût pourri, c'est-à-dire atteint par le botrytis est représenté par le trait discontinu référencé en 2.

Ce graphique illustre la consommation brute d'oxygène par le milieu dans un temps de mesure déterminé, la mesure étant effectuée après élimination des consommations d'oxygène parasite.

La laccase est un enzyme traceur du Botrytis qui est absente des vendanges saines et qui est présente dans les vendanges pourries. Elle a été choisie dans l'exemple ci-dessus en raison de ses qualités de solubilité et de relative stabilité et de spécificité. Dans le cas d'autres échantillons que le raisin, la laccase est également traceur du Botrytis par exemple sur les fraises ou sur d'autres produits.

La laccase peut en outre servir de traceur d'autres altérations que celles liées au Botrytis, sur d'autres produits que le raisin. Cependant d'autres traceurs ou marqueurs de BC tels que des enzymes du type glucose-oxydase ou Betaglucanase ou des corps chimiques tels qu de l'acide gluconique ou musiques peuvent etre utilisés.

La formule générale de la réaction est la suivante :

Substrat + O2      Laccase             Substrat oxyde
                   Echantillon

Le réactif utilisé englobe sous ce terme générique un substrat spécifique ou non, et une fonction inhibiteur de consommation d'oxygène parasite, le milieu étant ajusté vis à vis du PH.

Le substrat est de préférence un hydroxynaphtalène.
Le substrat peut également etre constitué par d'autres dihydroxy-naphtalènes et dérivés ayant des qualités de substrats spécifiques de la laccase et qui peuvent etre utilisés parce que plus stables, ou plus solubles ou plus sensibles.

De meme sans sortir du cadre de l'invention d'autres substrats ou réactifs permettant de déterminer la présence d'autres enzymes ou constituants chimiques que la laccase les dits enzymes ou constituants pouvant entrainer directement ou indirectement une variation de l'oxygène dissout peuvent etre utilisés.
Ce peut etre par exemple des substrats propres à d'autres enzymes que l'on veut détecter. Des enzymes externes rajoutés à l'échantillon créant une variation d'oxygène dissous à partir du constituant à choisir ou à mesurer.

L'inhibiteur de réaction de consommation d'oxygène parasite est du type spécifique ou non.
L'eau peut etre remplacée par d'autres diluants ou par d'autres solvants qui peuvent par exemple permettre d'extraire ou de solubiliser des éléments fixes sur les parties solides de l'échantillon.

La mesure de la consommation brute ou de la production d'oxygène directement par le milieu est effectuée au moyen d'électrodes à oxygène disposées dans le mélange réactif plus échantillon dans une chambre de mélange et de mesure comme il sera énoncé plus avant.

L'electrode à oxygène peut etre de type polarographique (de type electrode de CLARK ou de type KIMMICH KRUEZER) dans laquelle la différence de potentiel est d'origine externe, ou de type galvanique dans laquelle la différence de potentiel est d'origine interne (type MANCY ou MACKERETH ou BORKOWSKI par exemple).

La réduction d'oxygène à la cathode, constituée par un métal noble crée un courant électrique proportionnel à la quantité d'oxygène réduit.
La mesure de ce courant électrique permet d'estimer la pression partielle d'oxygène dans l'échantillon (P02) qui permet de déterminer l'oxygène dissout.

Les signaux électriques émis par l'électrode sont transmis à une centrale de calcul électronique comme énoncé plus avant.
L'installation de mesure selon l'invention comprend essentiellement un module mécanique et un module électronique doté d'une centrale de mesure des données et de moyens d'asservissement du module mécanique.

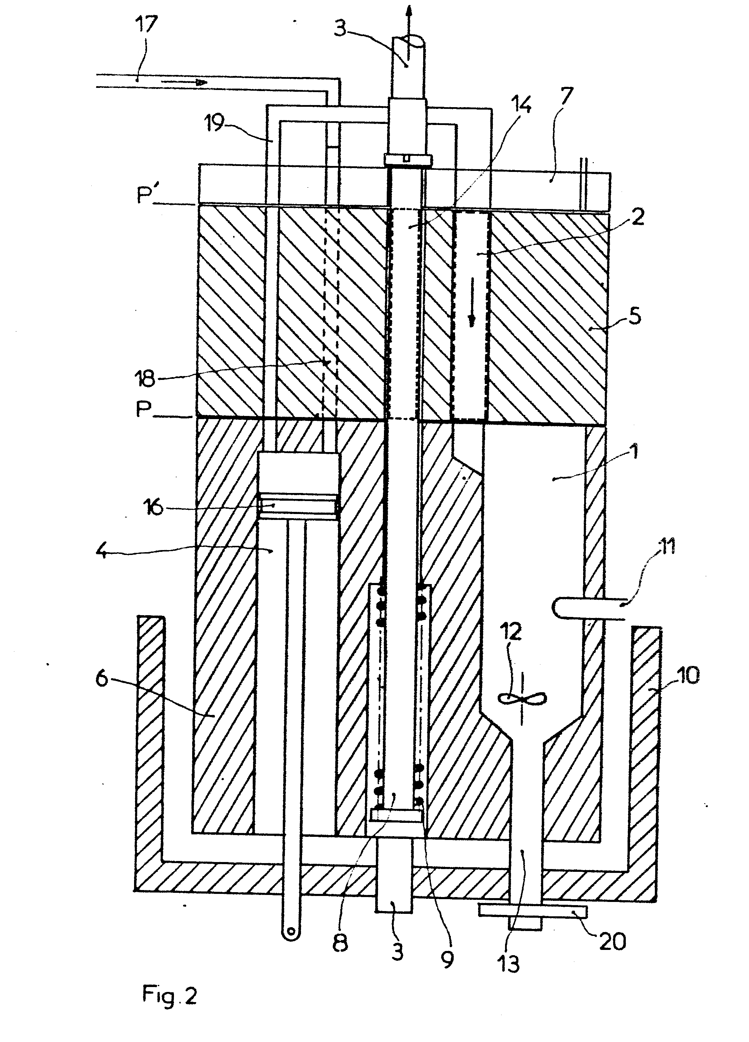

Le module mécanique (fig.2) comporte au moins une cuve de mesure 1 où s'effectue la réaction et dans laquelle plonge l'électrode à oxygène.
Le module mécanique comporte un dispositif 2 de prise et de distribution d'une dose d'échantillon en relation avec en amont une conduite 3 d'amenée du moût et en aval la cuve de mesure.
Ce module mécanique comporte également un dispositif 4 de prise et de distribution d'une dose de réactif, le dit dispositif étant également en relation avec la dite cuve de mesure 1.

L'ensemble de ces organes est commandé par des organes moteurs et par des moyens d'asservissement qui seront décrits plus en détail.

Le module mécanique réprésenté à la fig.2 est constitué par un distributeur 5 monté rotatif entre une partie inférieure 6 fixe et une partie supérieure fixe 7 autour d'un axe de fixation 8 coopérant avec un organe de blocage et avec un organe élastique 9 qui contribue à maintenir la partie inférieure 6 et la partie supérieure 7 en pression contre le distributeur 5.

L'ensemble du module mécanique est monté dans une enceinte 10 représentée schématiquement et qui reçoit un fluide chauffé en l'espèce de l'eau à une température régulée par un thermostat non représenté.

La température de l'eau qui peut être comprise entre 25 à 30° a pour objet de maintenir le module à une température permettant de réaliser les différentes mesures dans des conditions optimales.

La partie 6 fixe inférieure du module comprend la cuve de mesure 1. Dans la cuve de mesure est monté un capteur qui est une électrode spécifique à oxygène représentée schématiquement en 11 et un moyen d'agitation schématisé en 12 qui permet d'homogénéiser le milieu réactif, de mélanger l'oxygène et de renouveler le produit au contact avec l'électrode.

Le moyen d'agitation peut être mécanique ou magnétique.

Un conduit de vidange 13 en relation avec l'extérieur par un organe d'ouverture et de fermeture 20 commandé permet l'évacuation de la cuve de mesure qui est alimentée par le distributeur 5 et le prélèvement éventuel de l'échantillon.

Le distributeur 5 mobile entre les plans PP' est doté d'un conduit parallèle à l'axe 8 constituant le dispositif 2 de prise et de distribution d'une dose d'échantillon.

Le conduit 2 est alimenté par l'intermédiaire d'un conduit ménagé dans l'axe 8 le dit conduit étant en relation avec la conduite 3 d'arrivée du moût et d'acheminement de celui-ci vers son évacuation en sorte que dans une position représentée en pointillée et répertoriée par la référence 14 il soit alimenté en moût alors que par rotation de valeur de par exemple un quart de tour il vienne à la position correspondant à la référence 2 qui le met en communication avec la cuve 1.

L'alimentation en moût s'effectue au moyen d'une pompe non représentée.

Cette disposition permet de prélever à chaque rotation du distributeur un volume déterminé et fixe de l'échantillon.

La partie inférieure 6 est également dotée d'un dispositif référencé par 4 de prise et de distribution d'une dose de réactif.

Ce dispositif est constitué par une seringue dont le piston 16 est entrainé suivant un mouvement de va et vient dans une chambre en sorte de prélever une dose de réactif et de la pousser dans la cuve 1.

Un conduit 17 d'acheminement du réactif depuis une réserve non représentée est en relation avec la seringue par un conduit 18 du distributeur rotatif 5 lors de l'aspiration par mouvement de la seringue

Par rotation du distributeur, le conduit 17 n'étant plus en relation avec le conduit 18 un conduit 19 est mis en relation avec le dit conduit 18.

Le conduit 19 est en relation avec le conduit 2 de piegeage de l'échantillon lors du refoulement obtenu par mouvement de la seringue, ce refoulement s'effectuant après rotation du distributeur de la position 14 à la position de mise en contact avec la cuve 1.

L'échantillon de moût (ou d'autres produits) est prélevé directement sur la conduite 3 d'amenée par le conduit 2 ce qui permet d'obtenir une meilleur représentativité et reproductivité de l'échantillon à prélever.

Le réactif est poussé par la seringue à travers le conduit 2 vers la cuve 1 en sorte qu'il contribue à rincer le dit conduit ce qui permet d'expulser dans la cuve la totalité de l'échantillon prélevé.

Le module mécanique selon l'invention présente l'avantage d'être particulièrement simple et de permettre par une seule rotation du distributeur 5 la mise en communication dans la cuve de mesure de tous les éléments necessaires au dosage : l'échantillon, le réactif et l'air.

La rotation du distributeur est obtenue par des moyens connus à la portée de l'homme de l'art ainsi que le mouvement du piston 16 de la seringue.

Ces moyens du type moteur électrique et bielle manivelle ou came à excentrique sont commandés par un module électronique programmable.

Le fonctionnement du module mécanique de l'installation selon l'invention est le suivant :

A la position repos, le distributeur 5 est en position de prise d'échantillon et la seringue est remplie de réactif (position basse du piston 16).

Une pompe non représentée actionnée suivant une durée programmable envoie l'échantillon.

Par rotation du distributeur, l'échantillon est amené à la cuve 1 de mesure, la seringue actionnée par un organe moteur non représenté vide alors le réactif à travers le conduit 2 de prise d'échantillon dans la cuve 1.

L'agitation est actionnée un bref laps de temps après quoi la réaction et la mesure débutent.

Le distributeur revient à la position de départ avec mise en communication des conduits 17 et 18 et aspiration d'une dose en attente de réactif. La cuve de mesure est alors ouverte pour la vidange.

Les informations détectées par la sonde spécifique à oxygène sont transmise au module électronique de mesure et d'analyse des données d'exploitation.

Le module électronique commande les fonctions mécaniques, enregistre les ordres transmis ce qui permet d'asservir l'exécution des opéra-

tions depuis le quai de reception de la vendange, enregistre le signal du capteur, c'est-à-dire de l'electrode à oxygène (ou de tout autre capteur donnant un signal comparable) et procède à l'analyse du signal du capteur.

L'analyse du signal du capteur peut s'effectuer soit au niveau de la pression partielle d'oxygène dans l'échantillon ce qui permet de connaître la quantité d'oxygène dissous ou le pourcentage d'oxygène soit par la mesure de la vitesse de variation de l'oxygène.

Le module électronique comporte des fonctions de correction : correction des variations instantannées du signal par mesure d'une moyenne de vitesse sur un temps donné, correction de la dérive de l'électrode par différents systèmes possibles d'étalonnage et correction de la valeur initiale d'oxygène dissous en exprimant la vitesse de consommation d'oxygène ramenée à l'unité de concentration en oxygène dissous dans le milieu réactionnel.

Le module électronique est doté de moyens de traitement des informations qui peuvent comporter un système d'affichage en activité enzymatique exprimée en oxygène consommée par unité de temps et unité de volume d'echantillon ou exprimée suivant un classement modulable par l'utilisateur qui peut eêtre par exemple établi suivant quatre classes :

A = produit sain
B = produit peu atteint
C = produit atteint
D = produit très atteint.

Le module électronique peut également fre doté de moyens de sorties des informations soit vers un organe enregistreur ou une imprimante, soit vers une alarme, soit vers la commande pour asservissement du matériel aval, par exemple pour orienter les vannes à vendange et séparer les lots atteints des lots sains, soit pour régler des matériels de sulfitage ou de thermo-vinification ou autre.

**0095432**

Le procédé selon l'invention et l'installation mise en oeuvre sont particulièrement interessant dans l'application qui en est donnée à titre d'exemple non limitatif au moût de vendange mais il va de soi que comme énoncé elle peut être employé à la mesure comme défini plus avant de l'état sanitaire de tout matériau biologique.

La présente invention peut recevoir des aménagements et des variantes dans le domaine des équivalents techniques sans pour autant sortir du cadre du présent brevet.

REVENDICATIONS

R1/ Procédé de détermination et de mesure de l'état sanitaire d'un matériau biologique caractérisé en ce qu'il est fait usage de l'aptitude du parasite à générer par évolution de l'état sanitaire du matériau une oxydase qui est utilisée comme traceur, c'est-à-dire comme révélateur de l'état sanitaire, et en ce qu'un échantillon du dit matériau est mis en contact avec une dose du dit réactif en présence d'air ou d'un autre gaz et en ce que par mesure de la quantité d'oxygène consommée par unité de temps et de volume est déterminée l'absence ou la présence de la dite oxydase et l'importance de l'activité oxydasique et donc l'état sanitaire du dit matériau.

R2/ Procédé de mesure de l'état sanitaire d'un produit selon la revendication 1 caractérisé en ce que l'oxydase utilisée comme traceur est la laccase.

R3/ Procédé de mesure de l'état sanitaire d'un produit selon la revendication 1 et la revendication 2 caractérisé en ce que le réactif est un hydroxynaphtalène.

R4/ Procédé de mesure de l'état sanitaire d'un produit selon la revendication 1 caractérisé en ce que l'oxydase et le réactif correspondant sont choisis en fonction de la nature de l'affection sanitaire du produit à déterminer.

R5/ Installation de mesure permettant la mise en oeuvre du procédé selon la revendication 1 caractérisé en ce qu'elle comporte au moins une cuve de mesure (1) dans laquelle s'effectue la réaction, dotée de moyens (11) de mesure reliée avec une centrale électronique de mesure de l'activité oxydasique et de traitement des données, au moins un dispositif distributeur (5) doté de au moins un dispositif (2) de prise et de distribution d'un échantillon du matériau, le dit dispositif (2) étant en relation avec la dite cuve, et d'un dispositif (4) de prise et de distribution d'une dose de réactif le dit dispositif étant en communication avec la dite cuve (1) de mesure.

R6/ Installation selon la revendication précédente, caractérisée en ce que le dispositif (4) de distribution d'une dose de réactif est mis en communication avec le dispositif (2) de prise d'un échantillon pour déversement des produits dans la cuve de mesure.

R7/ Installation selon les revendications 5 et 6 caractérisée en ce qu'elle comporte un module mécanique dont une partie inférieure fixe (6) est dotée d'une cuve (1) de réaction et de mesure et d'un dispositif (4) de prise et de distribution d'une dose de réactif et dont une partie (5) ou distributeur mobile en rotation entre la dite partie inférieure fixe (6) et une partie supérieure (7), le dit distributeur étant doté d'un conduit (2) de prise d'une quantité de matériau échantillon en relation avec un conduit (3) de distribution et d'un conduit (18) de mise en communication du dispositif (4) de prise de réactif avec un conduit (17) d'amenée du dit réactif et en ce que par rotation du distributeur (5) entre la partie inférieure et la partie supérieure le conduit est mis en relation avec la cuve (1) et le dispositif (4) est mis en relation avec le conduit (2).

R8/ Installation selon les revendications 5 et 7 caractérisée en ce que le dispositif (4) est une seringue dont un piston (16) est mobile dans la partie inférieure du module mécanique.

Fig.1

Fig. 2

Office européen
des brevets

**RAPPORT DE RECHERCHE EUROPEENNE**

0095432
Numéro de la demande

EP 83 45 0014

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| X | CHEMICAL ABSTRACTS, vol. 82, no. 25, juin 1975, pages 375,376, no. 168804g, Columbus, Ohio, USA M. DUBERNET et al.: "Causes and consequences of oxygen consumption by grape musts" & VITIS 1974, 13(3), 233-244 * Abrégé en entier * | 1,2,4 | C 12 Q 1/26 G 01 N 33/14 |
| A | US-A-4 145 255 (J.G. SCHILLER et al.) | | |
| A | CHEMICAL ABSTRACTS, vol. 79, no. 9, 3 septembre 1973, page 248, no. 51804d, Columbus, Ohio, USA F. RADLER et al.: "Affinity for oxygen of polyphenol oxidase in grapes" & Z. LEBENSM.-UNTERS. FORSCH. 1973, 152(1), 38-41 | | |
| A | CHEMICAL ABSTRACTS, vol. 91, no. 23, 3 décembre 1979, page 479, no. 191311p, Columbus, Ohio, USA T. IVANOV: "Effect of oxygen on the composition and quality of must and white wine" & LOZAR. VINAR. 1979, 28(4), 31-39 | | |

---    -/-

**DOMAINES TECHNIQUES RECHERCHES (Int. Cl. 3)**

G 01 N
C 12 Q

Le présent rapport de recherche a été établi pour toutes les revendications

| Lieu de la recherche | Date d'achèvement de la recherche | Examinateur |
|---|---|---|
| LA HAYE | 05-09-1983 | GRIFFITH G. |

Office européen
des brevets

**RAPPORT DE RECHERCHE EUROPEENNE**

0095432
Numéro de la demande

EP  83 45 0014

Page  2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| A | CHEMICAL ABSTRACTS, vol. 89, no. 9, 28 août 1978, page 320, no. 74189c, Columbus, Ohio, USA J. LASZLO et al.: "The combination of molecular oxygen in grape must and young wine and its consequences" | | |
| | ----- | | |
| | | | **DOMAINES TECHNIQUES RECHERCHES (Int. Cl. 3)** |

Le présent rapport de recherche a été établi pour toutes les revendications

| Lieu de la recherche | Date d'achèvement de la recherche | Examinateur |
|---|---|---|
| LA HAYE | 09-09-1983 | GRIFFITH G. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82